# EUROPEAN PATENT APPLICATION

(11) **EP 4 122 454 A1**
(43) Date of publication of application: **25.01.2023**
(21) Application number: 21770418.8
(22) Date of filing: 04.02.2021
(51) Int. Cl.: A61K 31/00, A61K 31/714, A23L 33/00, A23L 33/10, A61K 33/00, A61K 33/34, A61P 39/00

(54) **ADDITIVE FOR DETOXIFICATION**

(30) Priority: 20.03.2020 BR 102020005629
(71) Applicant: Silva, Wellington Nascimbeni, 35160144 Ipatinga (BR)
(72) Inventor: Silva, Wellington Nascimbeni, 35160144 Ipatinga (BR)
(74) Representative: Lorente Berges, Ana
(86) International application number: PCT/BR2021/050051
(87) International publication number: WO 2021/184094

(57) **Abstract**

The present invention patent application relates essentially to an additive, the function of which is to enhance immunity, prevent various diseases and increase detoxification of the organism. The additive is composed of: 60 mcg of vitamin A; 4.5 mg of vitamin C; 1 mg of vitamin E; 3.4 mcg of selenium; 0.5 mg of copper; 0.7 mg of zinc; 0.23 mg of manganese; 3 mg of coenzyme 0.10; 5 mg of pycnogenol; 0.13 mg of vitamin B2; 1.6 mg of vitamin B3; 0.13 mg of vitamin B6; 24 mcg of folic acid; 0.24 mcg of vitamin B12; 2 mg of glutathione; 100 mg of leucine; 20 mg of isoleucine; 20 mg of valine; 50 mg of glycine; 50 mg of taurine; 300 mg of glutamine; 60 mg of acetylcysteine; 50 mg of cysteine; 100 mg of methionine; 0.5 mg of vitamin B5; 2 mg of lycopene; and 4 mg of resveratrol.

## Description

### INTRODUCTION:

1. The present patent application for the Privilege of Invention refers to an additive which function is to increase immunity, prevent various diseases, increase the detoxification of the organism.

2. Said product, which protection will be claimed in this report, was developed with the aim of filling deficiencies, alleviating difficulties and solving problems hitherto encountered by users of the sector, more precisely, the Biochemical and pharmaceutical sectors.

### FEATURES:

3. The chemical composition in question, presented through this report, is composed of: 60 mcg of vitamin A; 4.5 mg of vitamin C; 1 mg vitamin E; 3.4 mcg of selenium; 0.5 mg of copper; 0.7 mg of zinc; 0.23 mg manganese; 3 mg of coenzyme Q10; 5 mg of pycnogenol; 0.13 mg of vitamin B2; 1.6 mg of vitamin B3; 0.13 mg of Vitamin B6; 24 mcg of folic acid; 0.24 mcg of vitamin B12; 2 mg of Glutathione; 100mg of Leucine; 20 mg of Isoleucine; 20 mg of valine; 50 mg of Glycine; 50 mg of taurine; 300 mg of Glutamine; 60 mg of acetylcysteine; 50 mg Cysteine; 100 mg of Methionine; 0.5 mg of Vitamin B5; 2 mg of lycopene and 4 mg of resveratrol.

### FUNCTIONALITY:

4. Initially, the aforementioned chemical composition, described in this report, will be used in the Biochemistry and Pharmaceutical sectors.

5. This product is based on the principle of detoxification of the body, with minerals, vitamins and other compounds present in its formulation that are necessary for the detoxification process to occur in the body, that is, they all have the same function in the formula, that of help promote detoxification, an action that occurs through the activity of all components together.

6. It will act in the body's detoxification process, that is, helping to eliminate toxins from the body. The detoxification process is a chain of chemical reactions that occur in the body in order to make substances foreign to the body more hydrophilic and thus more easily eliminated from the body.

7. The purpose of this patent application is the development of a new food formula that will be able to obtain the result described for the purpose of this additive, which is to increase the body's detoxification process.

8. In research carried out in medical/scientific production databases, there is no result on what is being proposed.

9. Manufacturing will be done by combining twenty-seven components: 60 mcg of vitamin A; 4.5 mg of vitamin C; 1 mg vitamin E; 3.4 mcg of selenium; 0.5 mg of copper; 0.7 mg of zinc; 0.23 mg manganese; 3 mg of coenzyme Q10; 5 mg of pycnogenol; 0.13 mg of vitamin B2; 1.6 mg of vitamin B3; 0.13 mg of Vitamin B6; 24 mcg of folic acid; 0.24 mcg of vitamin B12; 2 mg of Glutathione; 100mg of Leucine; 20 mg of Isoleucine; 20 mg of valine; 50 mg of Glycine; 50 mg of taurine; 300 mg of Glutamine; 60 mg of acetylcysteine; 50 mg Cysteine; 100 mg of Methionine; 0.5 mg of Vitamin B5; 2 mg of lycopene and 4 mg of resveratrol.

### INNOVATION:

10. In general terms, said chemical composition represents a solution capable of obtaining the concentration with vitamins, minerals, amino acids and bioactive compounds.

11. The technical and functional advantage is that it is unique because it brings together all the vitamins, minerals and other substances that act in the body's detoxification process, helping a healthier life. As an economic and functional advantage, we can mention that it will not be necessary to ingest a large amount of food for the body to start the detoxification process more efficiently, which would save money and energy in the body.

### DESCRIPTION OF THE STATE OF THE TECHNIQUE:

12. During the development of the aforementioned chemical composition, numerous researches were carried out to identify the existence of possible prior art or similar compositions. Such surveys, however, did not point to the existence of any other composition with the same preponderant technical or functional characteristics.

13. Faced with this need and commercial opportunity, the aforementioned composition was created, more precisely with a combination of twenty-seven components: 60 mcg of vitamin A; 4.5 mg of vitamin C; 1 mg vitamin E; 3.4 mcg of selenium; 0.5 mg of copper; 0.7 mg of zinc; 0.23 mg manganese; 3 mg of coenzyme Q10; 5 mg of pycnogenol; 0.13 mg of vitamin B2; 1.6 mg of vitamin B3; 0.13 mg of Vitamin B6; 24 mcg of folic acid; 0.24 mcg of vitamin B12; 2 mg of Glutathione; 100mg of Leucine; 20 mg of Isoleucine; 20 mg of valine; 50 mg of Glycine; 50 mg of taurine; 300 mg of Glutamine; 60 mg of acetylcysteine; 50 mg Cysteine; 100 mg of Methionine; 0.5 mg of Vitamin B5; 2 mg of lycopene and 4 mg of resveratrol, forming a compound in a specific dilution to obtain the desired effect.

14. Therefore, in accordance with article 8 of the Industrial Property Law No. 9,279/96 and for all the aspects presented in this report, the object of this patent application deserves protection as a Privilege of Invention, which is now claimed.

## Claims

1. ADDITIVE FOR DETOXIFICATION - This is an additive to increase immunity, prevent various diseases, increase the detoxification of the body, **characterized by** the fact that it comprises: 60 mcg of vitamin A; 4.5 mg of vitamin C; 1 mg vitamin E; 3.4 mcg of selenium; 0.5 mg of copper; 0.7 mg of zinc; 0.23 mg manganese; 3 mg of coenzyme Q10; 5 mg of pycnogenol; 0.13 mg of vitamin B2; 1.6 mg of vitamin B3; 0.13 mg of Vitamin B6; 24 mcg of folic acid; 0.24 mcg of vitamin B12; 2 mg of Glutathione; 100mg of Leucine; 20 mg of Isoleucine; 20 mg of valine; 50 mg of Glycine; 50 mg of taurine; 300 mg of Glutamine; 60 mg of acetylcysteine; 50 mg Cysteine; 100 mg of Methionine; 0.5 mg of Vitamin B5; 2 mg of lycopene and 4 mg of resveratrol.
